# EUROPEAN PATENT SPECIFICATION

(11) **EP 3 058 932 B1**
(45) Date of publication and mention of the grant of the patent: **26.07.2023**
(21) Application number: 16155216.1
(22) Date of filing: 11.02.2016
(51) Int. Cl.: A61K 6/90

(54) **SILICONE ELASTOMER COMPOSITION FOR USE AS DENTAL IMPRESSION MATERIAL**
SILIKONELASTOMERZUSAMMENSETZUNG ZUR VERWENDUNG ALS ZAHNABDRUCKMATERIAL
COMPOSITION D'ÉLASTOMÈRE DE SILICONE POUR UTILISATION COMME MATÉRIAU D'EMPREINTE DENTAIRE

(30) Priority: 19.02.2015 JP 2015030613
(43) Date of publication of application: 24.08.2016
(73) Proprietor: Shin-Etsu Chemical Co., Ltd., Tokyo (JP)
(72) Inventor: YAMAZAKI, Toshio, Annaka-shi,, Gunma (JP); SUGAHARA, Hideki, Annaka-shi,, Gunma (JP)
(74) Representative: Ter Meer Steinmeister & Partner

(56) References cited:
- EP-A1- 1 361 253
- EP-A2- 2 165 693
- US-A1- 2011 281 237
- US-A1- 2012 083 549
- US-A1- 2013 267 628

## Description

### TECHNICAL FIELD

This invention relates to a silicone elastomer composition for use as a dental impression material as defined in claim 1.

### BACKGROUND ART

Dental impression materials have hitherto been made of, for example, agar, alginate, polysulfide rubber, polyether rubber or silicone elastomers. Silicone elastomers in particular are widely used because of their very precise dimensional stability.

With all such materials, the dentist forms a rubber mold by covering the dentition within the oral cavity with a composition in liquid or putty form, curing the composition at a normal temperature by a suitable curing mechanism, then removing the cured composition (rubber mold). Gypsum is cast into this rubber mold, then cured and subsequently removed from the mold to produce a gypsum model of the oral dentition. In conventional dental impression materials, a reinforcing filler such as carbon or silica is compounded with the base polymer in order to impart rubber strength, and so these materials are generally opaque. In addition, mold release agents for improving mold releasability, plasticizers for adjusting hardness and flowability, and various additives for conferring other properties often lack compatibility with the base polymer, causing the composition to become opaque.

When covering the dentition with an uncured dental impression material, minor adjustments are essential to carry out the positioning and air bubble removal which greatly impact the degree of perfection achieved in the rubber mold. However, because the dental impression material is opaque for the above reasons, visual confirmation is impossible, calling for expertise and a high level of skill in the dentist.

Also, dental impression materials are packed and distributed in cartridge-type containers, and it is common for dentists, when taking a dental impression, to discharge the impression material from the cartridge using a manually operated dispenser. When the dental impression material has too low a viscosity, covering the dentition is not easy. On the other hand, when the viscosity is too high, the material is difficult to manually dispense from the cartridge. Patent Document 6 discloses a silicone elastomer composition adapted for use as a dental impression material. However, Patent Document 6 does not disclose an organopolysiloxane as defined in item D of claim 1.

### CITATION LIST

Patent Document 1: JP-B H08-13732
Patent Document 2: JP 2731987
Patent Document 3: JP 2967236
Patent Document 4: JP 3928071
Patent Document 5: JP 4090536
Patent Document 6: US 2013/267628 A1
Patent Document 7: EP 2 165 693 A2
Patent Document 8: US 2011/281237 A1

### DISCLOSURE OF THE INVENTION

It is therefore an object of this invention to provide a silicone elastomer composition adapted for use as a dental impression material, which composition, prior to curing, exhibits a flowability suitable for taking a dental impression and, after curing, has an excellent mechanical strength--including tear strength and elongation--and moreover gives a cured product of high transparency.

The inventors have discovered that this object can be achieved by using a silicone elastomer composition which does not include reinforcing fillers such as silica in a range that diminishes transparency. They have also found that a silicone elastomer composition which ensures a sufficient strength as a dental impression material and has a flowability suitable for taking a dental impression can be obtained by using in combination a plurality of alkenyl group-containing organopolysiloxanes of differing molecular weights.

Accordingly, the invention provides a silicone elastomer composition adapted for use as a dental impression material as defined in claim 1. Preferred embodiments are defined in the dependent claims.

### ADVANTAGEOUS EFFECTS OF THE INVENTION

Dental impression materials made of the inventive silicone elastomer composition have a high transparency, enabling the dentist to work while visually checking directly, with the dental impression material covering the dentition, so as to, for example, make minor adjustments in the relative positions of the dentition and the dental impression material and remove air bubbles caught up during the procedure.

### DESCRIPTION OF THE PREFERRED EMBODIMENTS

The objects, features and advantages of the invention will become more apparent from the following detailed description.

### Component A

The diorganopolysiloxane serving as component A in the inventive silicone elastomer composition for use as a dental impression material has an average degree of polymerization of at least 300 and less than 3,000 and contains at least two silicon atom-bonded alkenyl groups per molecule.

Known diorganopolysiloxanes that are commonly used as base polymers in hydrosilylation-curable liquid silicone rubber compositions may be used as component A.

Component A has a viscosity at 23°C that is from 2 to 200 Pa·s, and preferably from 5 to 100 Pa·s. The viscosity can be measured with a rotational viscometer.

### Component B

The diorganopolysiloxane serving as component B has an average degree of polymerization of less than 300 and contains at least two silicon atom-bonded alkenyl groups per molecule.

Known diorganopolysiloxanes that are commonly used as base polymers in hydrosilylation-curable liquid silicone rubber compositions may be used as component B.

Component B has a viscosity at 23°C that is from 20 to 2,000 mPa·s, and more preferably from 100 to 1,000 mPa·s.

### Component C

The diorganopolysiloxane which is gum-like at room temperature and serves as component C has an average degree of polymerization of at least 3,000 and contains at least two silicon atom-bonded alkenyl groups per molecule.

Known diorganopolysiloxanes that are commonly used as base polymers in millable-type silicone rubber compounds may be used as component C.

The average degree of polymerization of component C is from 3,000 to 30,000, and preferably from 5,000 to 10,000.

The average degree of polymerization can be determined from the polystyrene-equivalent weight-average molecular weight obtained by gel permeation chromatography.

Components A, B and C are all alkenyl group-containing diorganopolysiloxanes, but they have mutually differing molecular weights (degrees of polymerization). Diorganopolysiloxanes represented by average compositional formula (I) below are generally preferred.

R¹ₐSiO_{(4-a)/2} (I)

Here, R¹ is a substituted or unsubstituted monovalent hydrocarbon group of 1 to 6 carbon atoms, illustrative examples of which include unsubstituted monovalent hydrocarbon groups such as alkyl groups (e.g., methyl, ethyl, propyl, butyl, pentyl and hexyl groups), alkenyl groups (e.g., vinyl, allyl, butenyl, pentenyl and hexenyl groups), and cyclohexyl, cyclohexenyl and phenyl groups; and substituted monovalent hydrocarbon groups (e.g., substituted alkyl groups) in which at least some portion of the hydrogen atoms on the foregoing unsubstituted monovalent hydrocarbon groups have been substituted with halogen atoms or cyano groups, such 3,3,3-trifluoropropyl and cyanomethyl groups. When there are a plurality of substituents, they may be the same or different, although it is critical that the molecule contain at least two alkenyl groups. In formula (I), the letter a is a number in the range of 1.9 to 2.4, and preferably 1.95 to 2.05. These diorganopolysiloxanes may each be linear or branched. Preferred diorganopolysiloxanes are linear organopolysiloxanes in which the main chain consists of repeated diorganopolysiloxane units (D units) and both ends of the molecular chain are capped with triorganosiloxy groups (M units). The silicon atom-bonded substituents are preferably methyl or phenyl groups. The two or more silicon atom-bonded alkenyl groups that must be contained on the molecule are preferably vinyl groups, these being present at the ends of the molecular chain or on side chains, with diorganopolysiloxanes having two such groups at both ends being preferred.

### Component D

The silicone resin serving as component D consists of units selected from among R₃SiO_{1/2} units (M units), SiO_{4/2.} units (Q units), R₂SiO_{2/2} units (D units) and RSiO_{3/2} units (T units). The combined amount of M units and Q units is at least 80 mol % of all constituent units, and the (number of moles of M units)/(number of moles of Q units) ratio is in the range of 0.5 to 1.5. Here, R is a monovalent hydrocarbon group of 1 to 6 carbon atoms, and at least two R groups per molecule are alkenyl groups.

Illustrative examples of R include unsubstituted monovalent hydrocarbon groups such as alkyl groups (e.g., methyl, ethyl, propyl, butyl, pentyl and hexyl groups), alkenyl groups (e.g., vinyl, allyl, butenyl, pentenyl and hexenyl groups), and cyclohexyl, cyclohexenyl and phenyl groups; and substituted monovalent hydrocarbon groups (e.g., substituted alkyl groups) in which at least some portion of the hydrogen atoms on the foregoing unsubstituted monovalent hydrocarbon groups have been substituted with halogen atoms or cyano groups, such as 3,3,3-trifluoropropyl and cyanomethyl groups. Although the plurality of R groups included in the silicone resin serving as component D may be the same or different, from the standpoint of compatibility with other ingredients in the silicone elastomer composition, it is preferable for at least 80 mol % of the R groups to be methyl groups. The reason is that when the compatibility of the ingredients in the composition worsens, the transparency of the cured form of the hydrosilylation-curable liquid silicone rubber composition decreases. The alkenyl groups are preferably vinyl groups. Here too, the reason is to maintain a good compatibility with the other ingredients.

In the silicone resin serving as component D, of the above four types of constituent units, the R₃SiO_{1/2} units (M units) and SiO_{4/4} units (Q units) are essential. To increase the hardness of the cured form of the hydrosilylation-curable liquid silicone rubber composition, it is critical that these two types of constituent units account for at least 80 mol % of all constituent units; they preferably account for at least 90 mol %, and more preferably 100 mol %, of all constituent units. R₂SiO_{2/2} (D units) and RSiO_{3/2} (T units) may or may not be included in component D.

At a (number of moles of M units)/(number of moles of Q units) ratio smaller than 0.5, the compatibility of the hydrosilylation-curable liquid silicone rubber composition with other ingredients worsens. At a ratio larger than 1.5, the hardness of the cured form of the hydrosilylation-curable liquid silicone rubber composition decreases. Therefore, the molar ratio of M units and Q units must be in the range of 0.5 to 1.5, and is preferably in the range of 0.7 to 1.2.

Illustrative examples of the silicone resin serving as component D include copolymers of vinyldimethylsiloxy groups and Q units, copolymers of vinyldimethylsiloxy groups/trimethylsiloxy groups and Q units, copolymers of vinyldimethylsiloxy groups/dimethylsiloxane and Q units, copolymers of vinyldimethylsiloxy groups/phenylsilsesquioxane and Q units, copolymers of vinyldimethylsiloxy groups/dimethylsiloxane/phenylsilsesquioxane and Q units, and copolymers of trimethylsiloxy groups/vinylmethylsiloxane and Q units.

Taking into account the flowability of the uncured composition and the rubber properties after curing, the inventive silicone elastomer composition for use as a dental impression material is obtained by compounding from 10 to 100 parts by weight of component B, from 10 to 200 parts by weight of component C and from 5.0 to 20 parts by weight of component D per 100 parts by weight of component A. In a composition where the amount of component B exceeds the above range, the composition when uncured has too high a flowability and does not remain on the dentition, making it impossible to form a rubber mold, in addition to which the cured composition is hard and brittle. In a composition where the amount of component B is too low, the viscosity of the uncured composition is too high, making it difficult to take a dental impression. In a composition where the amount of component C exceeds the above range, the viscosity of the uncured composition is too high, making it difficult to take a dental impression, and the cured form is soft and has surface tackiness, making it difficult to handle. In a composition where the amount of component C is too low, the uncured composition has too high a flowability and does not remain on the dentition, making it impossible to form a rubber mold. In a composition where the amount of component D exceeds the above range, the viscosity of the uncured composition is too high and the cured form is hard and brittle, making it unsuitable as a dental impression material.

The combined use of components B and C is important for achieving a good balance between the viscosity of the inventive silicone elastomer composition for use as a dental impression material when uncured and the hardness, tear strength and elongation after curing. The silicone elastomer composition of the invention satisfies all of the following rubber properties: when uncured, a viscosity at 23°C at least 100 Pa·s and less than 300 Pa·s; and, in the cured form, a hardness, as measured with a type A durometer, of at least 10 and not more than 30, and a tear strength and elongation at break, determined according to JIS K6249, of respectively at least 0.8 kN/m and not more than 2.0 kN/m and at least 150% and not more than 300%.

### Component E

The organohydrogenpolysiloxane serving as component E is an ingredient which forms crosslinkages by a hydrosilylation addition reaction with the alkenyl group-containing organopolysiloxanes of components A to D, thereby curing the composition. Component E contains at least two silicon atom-bonded hydrogen atoms per molecule, and preferably has the average compositional formula (II) below.

R²_{b}H_{c}SiO_{(4-b-c)/2} (II)

Here, R² is a substituted or unsubstituted monovalent hydrocarbon group of 1 to 6 carbon atoms, illustrative examples of which include unsubstituted monovalent hydrocarbon groups such as alkyl groups (e.g., methyl, ethyl, propyl, butyl, pentyl and hexyl groups) and cyclohexyl, cyclohexenyl and phenyl groups; and substituted monovalent hydrocarbon groups (e.g., substituted alkyl groups) in which at least some portion of the hydrogen atoms on the foregoing unsubstituted monovalent hydrocarbon groups have been substituted with halogen atoms or cyano groups, such as 3,3,3-trifluoropropyl and cyanomethyl groups. In formula (II), b is a positive number from 0.7 to 2.1, c is a positive number from 0.18 to 1.0, and b+c is from 0.8 to 3.0, with b being preferably from 0.8 to 2.0, c being preferably from 0.2 to 1.0, and b+c being preferably from 1.0 to 2.5.

The organohydrogenpolysiloxane serving as component E has a molecular structure which may be a linear, cyclic, branched or three-dimensional network structure. Preferred use can be made of an organohydrogenpolysiloxane which is liquid at room temperature and in which the number of silicon atoms on the molecule (or the degree of polymerization) is from about 2 to about 300, and especially from about 4 to about 200. The silicon atom-bonded hydrogen atoms may be present at the ends of the molecular chain or on side chains, or may be present at both the ends and on side chains.

Illustrative examples of the organohydrogenpolysiloxane serving as component E include methylhydrogenpolysiloxane capped at both ends with trimethylsiloxy groups, dimethylsiloxane/methylhydrogensiloxane copolymers capped at both ends with trimethylsiloxy groups, dimethylpolysiloxane capped at both ends with dimethylhydrogensiloxy groups, dimethylsiloxane/methylhydrogensiloxane copolymers capped at both ends with dimethylhydrogensiloxy groups, methylhydrogensiloxane/diphenylsiloxane copolymers capped at both ends with trimethylsiloxy groups, methylhydrogensiloxane/diphenylsiloxane/dimethylsiloxane copolymers capped at both ends with trimethylsiloxy groups, cyclic methylhydrogenpolysiloxanes, cyclic methylhydrogensiloxane/dimethylsiloxane copolymers, cyclic methylhydrogensiloxane/diphenylsiloxane/dimethylsiloxane copolymers, copolymers consisting of (CH₃)₂HSiO_{1/2} units and SiO_{4/2} units, and copolymers consisting of (CH₃)₂HSiO_{1/2} units, SiO_{4/2} units and (C₆H₅)SiO_{3/2} units.

In the rubber mold obtained after curing the silicone elastomer composition of the invention, to achieve a suitable strength, elongation and hardness, the amount of component E must be adjusted in such a way such that the number of silicon atom-bonded hydrogen atoms per silicon atom-bonded alkenyl group on components A, B, C and D is in the range of 0.5 to 5.0, and preferably from 1.0 to 2.0. At less than 0.5, a cured product that is soft and has surface tackiness results, whereas at more than 5.0, a cured product that is hard and brittle results.

### Component F

A known catalyst may be suitably used as the hydrosilylation catalyst serving as component F.

Illustrative examples include platinum-based catalysts such as platinum black, platinic chloride chloroplatinic acid, reaction products of chloroplatinic acid with monohydric alcohols, complexes of chloroplatinic acid and olefins, and platinum bisacetoacetate; and also palladium-based catalysts and rhodium-based catalysts. The hydrosilylation catalyst content may be treated as the amount of catalyst. This is from 0.5 tc 1,000 ppm, and preferably from 1 to 200 ppm, with respect to the combined amount of components A, B, C and D.

### Component G

The filler serving as component G in this invention is an ingredient for adjusting the viscosity and flowability of the inventive composition before curing and the strength after curing. It may be added within a range that does not detract from the transparency of the silicone elastomer composition for use as a dental impression material and the cured form thereof. Component G is included in a range of from 0 to 1 part by weight, although including 0.5 part by weight or less is preferred, and including no component G is more preferred. Above this range, the transparency of the silicone elastomer composition for use as a dental impression material, and of the cured form thereof, diminishes, in addition to which the viscosity of the composition when uncured rises, making it difficult to take a dental impression.

Illustrative examples of component G include reinforcing fillers such as fumed silica, wet finely powdered silica, crystalline silica, carbon black, red iron oxide, cerium oxide, titanium oxide, calcium carbonate, aluminum hydroxide and titanate esters; and fillers obtained by hydrophobizing the surface of these fillers with an organosilicon compound. Of these fillers, preferred use can be made of silicas that have little impact on the transparency when added to the silicone elastomer composition.

The filler has a 50% weight mean diameter, upon measurement of the particle size distribution by a laser diffraction method, of 1 µm or less, and preferably 0.1 µm or less. At a particle size greater than 1 µm, the transparency of the silicone elastomer composition for use as a dental impression material and of the cured form thereof diminishes.

In the rubber mold obtained after curing the inventive silicone elastomer composition for use as a dental impression material, the total light transmittance for a thickness of 2 mm is at least 90%. A transmittance of 90% or more is suitable for the dentist to carry out visual confirmation when covering the dentition with the dental impression material.

### Other Ingredients

In addition, to ensure a good pot life, the inventive silicone elastomer composition for use as a dental impression material may also have added thereto a hydrosilylation retarder, illustrative examples of which include polyfunctional alkenyl compounds such as 1,3,5,7-tetramethyl-1,3,5,7-tetravinylcyclotetrasiloxane and 1,3,5-triallyl-1,3,5-triazinane-2,4,6-(1H, 3H, 5H)-trione (triallyl isocyanurate); and acetylene alcohol derivatives such as 1-ethynylcyclohexanol and 3,5-dimethyl-1-hexyn-3-ol.

The inventive silicone elastomer composition for use as a dental impression material may also include, as crosslinking aids: silicon atom-bonded alkenyl group-containing organopolysiloxanes other than the alkenyl group-containing organopolysiloxanes of components A, B and C and the silicone resin of component D, alkoxysilyl group-containing alkoxysilane compounds, silane coupling agents, and titanium- or zirconium-based condensation catalysts, insofar as doing so does not detract from the advantageous effects of the invention. In addition, dyes, pigments, mold parting agents, hydrophilizing agents may also be included, provided that these do not detract from the advantageous effects of the invention. Such optional ingredients may be used singly or two or more may be used together.

The inventive silicone elastomer composition for use as a dental impression material may be prepared by using an ordinary mixing stirrer, kneader to dissolve, mix together and uniformly disperse the above ingredients.

No particular limitation is imposed on the method of curing the inventive silicone elastomer composition for use as a dental impression material. Suitable use can be made of known methods, such as molding the composition and then leaving it to stand at room temperature, molding the composition within the oral cavity of the patient and leaving it to stand in this manner, or molding the composition and then heating it at 50 to 200°C.

### EXAMPLES

Amounts of compounded ingredients are given in parts by weight. Weight-average molecular weights are polystyrene-equivalent values obtained by gel permeation chromatography.

### Working Examples 1 to 7 and Comparative Examples 1 to 6

### Working Examples 2-7 are not according to the claimed invention.

Tables 1 and 2 show the respective amounts in which the following ingredients were compounded in each example of a silicone elastomer composition for use as a dental impression material: (A) high-viscosity alkenyl group-containing organopolysiloxane, (B) low-viscosity alkenyl group-containing organopolysiloxane, (C) gum-like alkenyl group-containing organopolysiloxane, (D) silicone resin, (E) organohydrogenpolysiloxane, (F) hydrosilylation catalyst, (G) filler, and (H) hydrosilylation retarder. The compositions were uniformly mixed and stirred, then defoamed under reduced pressure and the viscosity was measured with a rotational viscometer.

A polyethylene terephthalate (PET) liner and a 2 mm thick retainer were stacked onto a pressing plate and the above silicone elastic composition for use as a dental impression material was cast into the retainer, following which another PET liner and another pressing plate were additionally stacked on top thereof and the composition was molded under pressure at room temperature (23°C) for 5 minutes. The cured material was removed together with the PET liners from between the two pressing plates, and the PET liners were peeled away, giving a transparent sheet of silicone elastomer having a thickness of 2 mm. The rubber hardness, tear strength, elongation at break, and total light transmittance of the sheets thus obtained in the respective examples were measured in accordance with JIS K6249.

The materials used in the Working Examples and Comparative Examples were as follows.

### (A) High-Viscosity Alkenyl Group-Containing Organopolysiloxane

| | |
|---|---|
| A-1 | Dimethylpolysiloxane capped at both ends with dimethylvinylsiloxy groups |
| | Weight-average degree of polymerization: about 1,100 (weight-average molecular weight, about 80,000) |
| | Viscosity, about 100 Pa·s |
| A-2 | Dimethylpolysiloxane capped at both ends with dimethylvinylsiloxy groups |
| | Weight-average degree of polymerization: about 450 (weight-average molecular weight, about 33,000) |
| | Viscosity, about 5 Pa·s |
| A-3 | Vinylmethylsiloxane/dimethylsiloxane copolymer capped at both ends with trimethylvinylsiloxy groups |
| | Weight-average degree of polymerization: about 750 (weight-average molecular weight, about 56,000) |
| | Viscosity, about 30 Pa·s |
| | Number of alkenyl groups per molecule, 5.3 |

### (B) Low-Viscosity Alkenyl Group-Containing Organopolysiloxane

| | |
|---|---|
| B-1 | Dimethylpolysiloxane capped at both ends with dimethylvinylsiloxy groups |
| | Weight-average degree of polymerization: about 180 (weight-average molecular weight, about 13,000) |
| | Viscosity, about 600 mPa·s |
| B-2 | Dimethylpolysiloxane capped at both ends with dimethylvinylsiloxy groups |
| | Weight-average degree of polymerization: about 70 (weight-average molecular weight, about 5,000) |
| | Viscosity, about 100 mPa·s |
| B-3 | Vinylmethylsiloxane/dimethylsiloxane copolymer capped at both ends with trimethylvinylsiloxy groups |
| | Weight-average degree of polymerization: about 180 (weight-average molecular weight, about 13,000) |
| | Viscosity, about 700 mPa·s |
| | Number of alkenyl groups per molecule, 2.7 |

### (C) Gum-Type Alkenyl Group-Containing Organopolysiloxane

| | |
|---|---|
| C-1 | Vinylmethylsiloxane/dimethylsiloxane copolymer capped at both ends with dimethylvinylsiloxy groups |
| | Weight-average degree of polymerization: about 8,000 (weight-average molecular weight, about 600,000) |
| | Number of alkenyl groups per molecule, 10 |
| C-2 | Dimethylpolysiloxane capped at both ends with dimethylvinylsiloxy groups |
| | Weight-average degree of polymerization: about 8,000 (weight-average molecular weight, about 600,000) |

### (D) Silicone Resin

| | |
|---|---|
| D-1 | Copolymer of (CH₃)₃SiO_{1/2} units, (CH₂=CH)(CH₃)₂SiO_{1/2} units and SiO_{4/2} units |
| | Molar ratio: (CH₃)₃SiO_{1/2}/(CH₂=CH)(CH₃)₂SiO_{1/2/}SiO_{4/2} = 40/10/50 |
| | Number of alkenyl groups per molecule, 4.2 (weight-average molecular weight = 3,000) |

### (E) Organohydrogenpolysiloxane

| | |
|---|---|
| E-1: | Dimethylsiloxane/methylhydrogensiloxane copolymer capped at both ends with dimethylhydrogensiloxy groups |
| | Viscosity: 12 mPa·s |

| | |
|---|---|
| | Number of SiH groups per molecule, 8 (SiH group content, 0.006 mol/g) |
| E-2: | Dimethylsiloxane/methylhydrogensiloxane copolymer capped at both ends with trimethylsiloxy groups |
| | Viscosity: 45 mPa·s |
| | Number of SiH groups per molecule, 45 (SiH group content, 0.011 mol/g) |

### (F) Hydrosilylation Catalyst

| | |
|---|---|
| F-1: | B-1 solution of Pt-divinyltetramethyldisiloxane complex Pt concentration, 1 wt % |

### (G) Filler

| | |
|---|---|
| G-1: | Aerosil R8200 (Nippon Aerosil Co., Ltd.), a surface-treated dry silica |
| | Specific surface area, 160±25 m²/g; average primary particle size, 12 nm |
| G-2: | Crystalite 5X (Tatsumori, Ltd.) a crystalline silica |
| | Average primary particle size, 1.5 µm |

### (H) Hydrosilylation Retarder

| | |
|---|---|
| H-1: | 1,3,5,7-Tetramethyl-1,3,5,7-tetravinylcyclotetrasiloxane |

**Table 1**

| Amount (pbw) | Working Example | | | | | | |
|---|---|---|---|---|---|---|---|
| | 1 | 2 | 3 | 4 | 5 | 6 | 7 |
| A-1 | 100.0 | 100.0 | | | | 100.0 | 100.0 |
| A-2 | | | 100.0 | 100.0 | | | |
| A-3 | | | | | 100.0 | | |
| B-1 | 50.0 | 50.0 | 10.0 | | | | 50.0 |
| B-2 | | | | 20.0 | 20.0 | | |
| B-3 | | | | | | 10.0 | |
| C-1 | 40.0 | 40.0 | 100.0 | 150.0 | | | 40.0 |
| C-2 | | | | | 60.0 | 50.0 | |
| D-1 | 5.0 | | | | | | |
| E-1 | 6.0 | | 3.5 | | | 2.0 | |
| E-2 | | 2.0 | | 3.0 | 8.0 | | 2.0 |
| F-1 | 1.0 | 1.0 | 1.0 | 1.3 | 0.9 | 0.8 | 1.0 |
| G-1 | | | | | | | 0.5 |
| G-2 | | | | | | | |
| H-1 | 0.10 | 0.10 | 0.10 | 0.13 | 0.09 | 0.08 | 0.10 |
| Molar ratio in reaction* | 2.0 | 1.9 | 1.8 | 1.8 | 4.8 | 0.6 | 1.9 |
| Viscosity (Pa·s) | 210 | 190 | 250 | 290 | 120 | 240 | 250 |
| Total light transmittance (%) | 96 | 98 | 94 | 93 | 98 | 98 | 90 |
| Hardness (Durometer type A) | 25 | 20 | 25 | 28 | 30 | 10 | 20 |
| Tear strength (kN/m) | 1.3 | 0.9 | 0.9 | 0.8 | 1.0 | 0.8 | 1.0 |
| Elongation at break (%) | 180 | 210 | 250 | 230 | 160 | 150 | 200 |

**Table 2**

| Amount (pbw) | Comparative Example | | | | | |
|---|---|---|---|---|---|---|
| | 1 | 2 | 3 | 4 | 5 | 6 |
| A-1 | 100.0 | 100.0 | 100.0 | 100.0 | 100.0 | 100.0 |
| A-2 | | | | | | |
| A-3 | | | | | | |
| B-1 | 50.0 | 50.0 | 120.0 | 50.0 | 5.0 | 50.0 |
| B-2 | | | | | | |
| B-3 | | | | | | |
| C-1 | 40.0 | 40.0 | 40.0 | 250.0 | 40.0 | 5.0 |
| C-2 | | | | | | |
| D-1 | | | | | | |
| E-1 | | | | | 1.7 | 3.6 |
| E-2 | 2.0 | 2.0 | 3.7 | 2.5 | | |
| F-1 | 1.0 | 1.0 | 1.3 | 2.0 | 0.6 | 0.8 |
| G-1 | 5.0 | | | | | |
| G-2 | | 0.5 | | | | |
| H-1 | 0.10 | 0.10 | 0.13 | 0.2 | 0.06 | 0.08 |
| Molar ratio in reaction* | 1.9 | 1.9 | 1.8 | 1.6 | 1.8 | 1.8 |
| Viscosity (Pa·s) | 450 | 200 | 90 | 350 | 320 | 50 |
| Total light transmittance (%) | 85 | 75 | 98 | 91 | 95 | 99 |
| Hardness (Durometer type A) | 35 | 20 | 28 | 15 | 13 | 28 |
| Tear strength (kN/m) | 5.3 | 0.9 | 0.9 | 0.5 | 0.6 | 0.8 |
| Elongation at break (%) | 350 | 200 | 150 | 270 | 280 | 120 |

| | | | | | | |
|---|---|---|---|---|---|---|
| *(Number of silicon atom-bonded alkenyl groups in components A, B, C and D)/(number of silicon atom-bonded hydrogen atoms in component E) | | | | | | |

Working Example 1, which satisfies the range of the invention, provides a highly transparent dental impression material which, prior to curing, has a viscosity suitable for taking a dental impression and, after curing, has an excellent tear strength and elongation at break.

In Comparative Example 1 having an amount of component G which falls outside the range of the invention, the transparency is low and the viscosity of the composition is too high, resulting in poor workability when taking a dental impression. In Comparative Example 2 having an average particle size for component G which falls outside the range of the invention, the transparency worsens.

In Comparative Example 3, wherein the amount of component B exceeds the upper limit of the invention, and in Comparative Example 6, wherein the amount of component C falls below the lower limit of the invention, the viscosity of the composition is too low, resulting in a poor workability when taking a dental impression.

In Comparative Example 5, wherein the amount of component B falls below the lower limit of the invention, and in Comparative Example 4, wherein the amount of component C exceeds the upper limit of the invention, the viscosity of the composition is too high, resulting in a poor workability when taking a dental impression, in addition to which the cured composition has a low tear strength.

The inventive silicone elastomer composition of the invention makes it possible to provide a dental impression material that achieves a high transparency. When taking a dental impression using this composition, the high transparency enables the dentist to work while visually checking the procedure directly so as to, for example, make minor adjustments in the relative positions of the dentition and the dental impression material and remove bubbles that have been caught up. In addition to being highly useful to the dentist, this helps shorten the time required to take a dental impression and therefore is beneficial to the patient as well.

## Claims

1. A silicone elastomer composition adapted for use as a dental impression material, which provides a rubber mold having the total light transmittance for a thickness of 2 mm of at least 90%, a hardness, as measured with a type A durometer, of at least 10 and not more than 30, a tear strength determined according to JIS K6249 of at least 0.8 kN/m and not more than 2.0 kN/m and an elongation at break determined according to JIS K6249 of at least 150% and not more than 300%, after curing said silicone elastomer composition,
the composition comprising:
(A) 100 parts by weight of a diorganopolysiloxane has an average degree of polymerization of at least 300 and less than 3,000 and contains at least two silicon atom-bonded alkenyl groups per molecule and has a viscosity at 23°C of from 2 to 200 Pa·s,
(B) from 10 to 100 parts by weight of a diorganopolysiloxane which has an average degree of polymerization of less than 300 and contains at least two silicon atom-bonded alkenyl groups per molecule and has a viscosity at 23°C of from 20 to 2,000 mPa·s,
(C) from 10 to 200 parts by weight of a diorganopolysiloxane which has an average degree of polymerization of 3,000 to 30,000, contains at least two silicon atom-bonded alkenyl groups per molecule and is gum-like at room temperature,
(D) from 5.0 to 20 parts by weight of an organopolysiloxane which consists of units selected from the group consisting of R₃SiO_{1/2} units (M units), SiO_{4/2} units (Q units), R₂SiO_{2/2} units (D units) and RSiO_{3/2} units (T units), contains M units and Q units as essential constituent units, has a combined amount of M units and Q units that is at least 80 mol % of all constituent units, and has a (number of moles of M units) / (number of moles of Q units) ratio in the range of 0.5 to 1.5 wherein R is a monovalent hydrocarbon group of 1 to 6 carbon atoms, and at least two R groups per molecule are alkenyl groups,
(E) an organohydrogenpolysiloxane which contains at least two silicon atom-bonded hydrogen atoms per molecule, in an amount such that the number of silicon atom-bonded hydrogen atoms per silicon atom-bonded alkenyl groups on components A, B, C and D is from 0.5 to 5.0,
(F) a hydrosilylation catalyst in an amount of from 0.5 to 1,000 ppm with respect to the combined weight of components A to E, and
(G) from 0 to 1 part by weight of a filler having an average particle size, measured as described in the description, of not more than 1 µm;
which composition has a viscosity at 23°C of at least 100 Pa-s and less than 300 Pa·s.

2. The silicone elastomer composition of claim 1, wherein the diorganopolysiloxanes serving as components A, B and C have average compositional formula (I) below
R¹ₐSiO_{(4-a)/2} (I)
wherein R¹ is a substituted or unsubstituted monovalent hydrocarbon group of 1 to 6 carbon atoms, and the letter a is a number in the range of 1.9 to 2.4.

3. The silicone elastomer composition of claim 1 or 2, wherein said component A has a viscosity at 23°C of from 5 to 100 Pa-s, said component B has a viscosity at 23°C of from 100 to 1,000 mPa·s and C has an average degree of polymerization of 5,000 to 10,000.

4. The silicone elastomer composition of any one of claim 1 to 3, which contains from 50 to 100 parts by weight of said component B, from 50 to 100 parts by weight of said component C and from 0 to 10 parts by weight of said component D per 100 parts by weight of said component A.

## Patentansprüche

1. Silikonelastomer-Zusammensetzung, die zur Verwendung als Zahnabdruckmaterial geeignet ist, die eine Kautschukform bereitstellt, die nach dem Härten der Silikonelastomer-Zusammensetzung eine Gesamtlichtdurchlässigkeit für eine Dicke von 2 mm von mindestens 90%, eine mit einem Durometer vom Typ A gemessene Härte von mindestens 10 und nicht mehr als 30, eine gemäß JIS K6249 bestimmte Reißfestigkeit von mindestens 0,8 kN/m und nicht mehr als 2,0 kN/m und eine gemäß JIS K6249 bestimmte Bruchdehnung von mindestens 150% und nicht mehr als 300% aufweist,
wobei die Zusammensetzung umfasst:
(A) 100 Gewichtsteile eines Diorganopolysiloxans, das einen durchschnittlichen Polymerisationsgrad von mindestens 300 und weniger als 3.000 aufweist und das mindestens zwei an ein Siliciumatom gebundene Alkenylgruppen pro Molekül enthält und eine Viskosität bei 23°C von 2 bis 200 Pa·s aufweist,
(B) 10 bis 100 Gewichtsteile eines Diorganopolysiloxans, das einen durchschnittlichen Polymerisationsgrad von weniger als 300 aufweist und mindestens zwei an ein Siliciumatom gebundene Alkenylgruppen pro Molekül enthält und eine Viskosität bei 23°C von 20 bis 2.000 mPa·s aufweist,
(C) 10 bis 200 Gewichtsteile eines Diorganopolysiloxans, das einen durchschnittlichen Polymerisationsgrad von 3.000 bis 30.000 aufweist, mindestens zwei an ein Siliciumatom gebundene Alkenylgruppen pro Molekül enthält und bei Raumtemperatur gummiartig ist,
(D) 5,0 bis 20 Gewichtsteile eines Organopolysiloxans, das aus Einheiten besteht, die ausgewählt sind aus der Gruppe bestehend aus R₃SiO_{1/2}-Einheiten (M-Einheiten), SiO_{4/2}-Einheiten (Q-Einheiten), R₂SiO_{2/2}-Einheiten (D-Einheiten) und RSiO_{3/2}-Einheiten (T-Einheiten), M-Einheiten und Q-Einheiten als wesentliche konstituierende Einheiten enthält, eine kombinierte Menge an M-Einheiten und Q-Einheiten aufweist, die mindestens 80 Mol-% aller konstituierenden Einheiten ausmacht, und ein Verhältnis (Molzahl der M-Einheiten)/(Molzahl der Q-Einheiten) im Bereich von 0,5 bis 1,5 aufweist, wobei R eine einwertige Kohlenwasserstoffgruppe mit 1 bis 6 Kohlenstoffatomen ist und mindestens zwei R-Gruppen pro Molekül Alkenylgruppen sind,
(E) ein Organohydrogenpolysiloxan, das mindestens zwei an ein Siliciumatom gebundene Wasserstoffatome pro Molekül enthält, in einer solchen Menge, dass die Anzahl der an ein Siliciumatom gebundenen Wasserstoffatome pro an ein Siliciumatom gebundene Alkenylgruppen der Komponenten A, B, C und D 0,5 bis 5,0 beträgt,
(F) einen Hydrosilylierungskatalysator in einer Menge von 0,5 bis 1.000 ppm, bezogen auf das Gesamtgewicht der Komponenten A bis E, und
(G) 0 bis 1 Gewichtsteil eines Füllstoffs mit einer durchschnittlichen Teilchengröße, gemessen wie in der Beschreibung beschrieben, von nicht mehr als 1 µm;
wobei die Zusammensetzung eine Viskosität bei 23°C von mindestens 100 Pa s und weniger als 300 Pa·s aufweist.

2. Silikonelastomer-Zusammensetzung nach Anspruch 1, wobei die als Komponenten A, B und C dienenden Diorganopolysiloxane die folgende durchschnittliche Zusammensetzungsformel (I) aufweisen
R¹ₐSiO_{(4-a)/2} (I)
worin R¹ eine substituierte oder unsubstituierte einwertige Kohlenwasserstoffgruppe mit 1 bis 6 Kohlenstoffatomen ist und der Buchstabe a eine Zahl im Bereich von 1,9 bis 2,4 ist.

3. Silikonelastomer-Zusammensetzung nach Anspruch 1 oder 2, wobei die Komponente A eine Viskosität bei 23°C von 5 bis 100 Pa·s aufweist, die Komponente B eine Viskosität bei 23°C von 100 bis 1.000 mPa·s aufweist und C einen durchschnittlichen Polymerisationsgrad von 5.000 bis 10.000 aufweist.

4. Silikonelastomer-Zusammensetzung nach einem der Ansprüche 1 bis 3, die 50 bis 100 Gewichtsteile der Komponente B, 50 bis 100 Gewichtsteile der Komponente C und 0 bis 10 Gewichtsteile der Komponente D pro 100 Gewichtsteile der Komponente A enthält.

## Revendications

1. Composition d'élastomère de silicone adaptée pour une utilisation comme matériau d'empreinte dentaire, qui fournit un moule en caoutchouc ayant la transmittance totale de la lumière pour une épaisseur de 2 mm d'au moins 90 %, une dureté, lorsqu'elle est mesurée avec un duromètre de type A, d'au moins 10 et non supérieure à 30, une résistance au déchirement déterminée conformément à la norme JIS K6249 d'au moins 0,8 kN/m et non supérieure à 2,0 kN/m et un allongement à la rupture déterminé conformément à la norme JIS K6249 d'au moins 150 % et non supérieur à 300 %, après durcissement de ladite composition d'élastomère de silicone,
la composition comportant :
(A) 100 parties en poids d'un diorganopolysiloxane qui a un degré de polymérisation moyen d'au moins 300 et inférieur à 3 000 et contient au moins deux groupes alcényle liés à des atomes de silicium par molécule et a une viscosité à 23 °C de 2 à 200 Pa·s,
(B) de 10 à 100 parties en poids d'un diorganopolysiloxane qui a un degré de polymérisation moyen inférieur à 300 et contient au moins deux groupes alcényle liés à des atomes de silicium par molécule et a une viscosité à 23 °C de 20 à 2 000 Pa·s,
(C) de 10 à 200 parties en poids d'un diorganopolysiloxane qui a un degré de polymérisation moyen de 3 000 à 30 000, contient au moins deux groupes alcényle liés à des atomes de silicium par molécule et est analogue à une gomme à température ambiante,
(D) de 5,0 à 20 parties en poids d'un organopolysiloxane qui est constitué de motifs choisis parmi le groupe constitué de motifs R₃SiO_{1/2} (motifs M), de motifs SiO_{4/2} (motifs Q), de motifs R₂SiO_{2/2} (motifs D) et de motifs RSiO_{3/2} (motifs T), contient des motifs M et des motifs Q en tant que motifs constitutifs essentiels, a une quantité combinée de motifs M et de motifs Q qui est au moins de 80 % en moles de tous les motifs constitutifs, et a un rapport (nombre de moles de motifs M )/(nombre de moles de motifs Q) dans l'intervalle de 0,5 à 1,5, dans lequel R est un groupe hydrocarbure monovalent de 1 à 6 atomes de carbone, et au moins deux groupes R par molécule sont des groupes alcényle,
(E) un organohydrogénopolysiloxane qui contient au moins deux atomes d'hydrogène liés à des atomes de silicium par molécule, en une quantité telle que le nombre d'atomes d'hydrogène liés à des atomes de silicium par groupes alcényle liés à des atomes de silicium sur les composants A, B, C et D est de 0,5 à 5,0,
(F) un catalyseur d'hydrosilylation en une quantité de 0,5 à 1 000 ppm par rapport au poids combiné des composants A à E, et
(G) de 0 à 1 partie en poids d'une charge ayant une taille de particule moyenne, mesurée tel que décrit dans la description, non supérieure à 1 µm ;
laquelle composition a une viscosité à 23° C d'au moins 100 Pa·s et inférieure à 300 Pa·s.

2. Composition d'élastomère de silicone selon la revendication 1, dans laquelle les diorganopolysiloxanes servant de composés A, B et C ont la formule de composition moyenne (I) ci-dessous :
R¹ₐSiO_{(4-a)/2} (I)
dans laquelle R¹ est un groupe hydrocarbure monovalent substitué ou non substitué de 1 à 6 atomes de carbone, et la lettre est un nombre dans l'intervalle de 1,9 à 2,4.

3. Composition d'élastomère de silicone selon la revendication 1 ou 2, dans laquelle ledit composant A a une viscosité à 23 °C de 5 à 100 Pa·s, ledit composant B a une viscosité à 23 °C de 100 à 1 000 mPa·s et C a un degré de polymérisation moyen de 5000 à 10 000.

4. Composition d'élastomère de silicone selon l'une quelconque des revendications 1 à 3, qui contient de 50 à 100 parties en poids dudit composant B, de 50 à 100 parties en poids dudit composant C et de 0 à 10 parties en poids dudit composant D pour 100 parties en poids dudit composant A.
